# EUROPEAN PATENT APPLICATION

(11) **EP 1 918 717 A1**
(43) Date of publication of application: **07.05.2008**
(21) Application number: 06022979.6
(22) Date of filing: 04.11.2006
(51) Int. Cl.: G01N 33/72, G01N 33/68

(54) **Method for measuring the levels of different target substances in a patient sample**

(71) Applicant: Olympus Life and Material Science Europa GmbH, 20097 Hamburg (DE)
(72) Inventor: Gunzer, Gerhard, Dr., Ennis Co. Clare (IE); O'Mahony, Sean, Clarecastle Co. Clare (IE); Pfuetzner, Ludwig, Dr., Ennis Co. Clare (IE)
(74) Representative: Emmel, Thomas

(57) **Abstract**

Method for measuring the levels of different target substances in a patient sample, especially a body fluid sample, in which before presentation of the test results to the user, it is evaluated for each target substance, whether or not the corresponding test result is influenced by an interfering substance, for example interference by hemolysis, icterus, or lipemia, in the patient sample and an appropriate flag, relative to the detected interfering substance, is assigned to those tests which may be influenced by the interfering substance and then the test results and possible flags are presented together to the user.

## Description

The invention relates to a method for screening a number of different substances in a patient sample.

The routine clinical chemistry profile obtained by determination of a number of different target substances is one essential element of the patient assessment. By a screening a physician can determine the presence of different disease states and additionally can use the data obtained to predict a potential problem before the patient shows more advanced problematic symptoms.

In a routine screening one can choose between a number of different target substances to be measured depending on the medical history and complaint of the patient. The target substances can be of different nature and include any metabolic substance whose concentration is characteristic for a certain metabolic status.

Typical target substances are amylase, lipase, cholesterol, glucose, creatinine, albumine, ferritin, calcium etc. to cite only some examples. Usually for screening of different target substances, test samples are prepared by mixing defined portions of the patient sample in separate vessels with diagnostic reagents each specific for one of the different target substances to be analysed.

The reagents are chosen to create an optical signal depending on the level of the respective target substance which can be measured photometrically.

One problem is that the patient sample can include chromatic substances which interfere with measurement and can thus produce errors in the results. Chromatic substances which typically can be present in a patient sample are haemoglobin (Hemolysis) and bilirubin (Icterus) and substances in relation with Lipemia (turbidity) like lipids, lipoproteins as well as protein precipitates. These substances absorb strongly either in the ultraviolet or visible portion of the spectrum. Since the methods in question employ optical detection means, the chromatic substances may interfere with the results of the screening.

For that reason known methods provide that the patient sample is checked for the possible presence of especially the mentioned interfering chromatic substances. Historically in most laboratories a visual inspection of the patient sample by specialised persons is performed to establish a chromatic interference status.

Alternatively the concentration of interfering substances can be qualitatively or semiquantitatively estimated by optical tests on automated analysers. If it is estimated that a patient sample includes one or more of the interfering chromatic substances, the interfering substance in question and the potential level of interference for individual tests is marked on the patient's record..

Table 1 shows typical markings which can be used to identify presence and concentration of an interfering substance on a record depending on the result of e.g. optical estimation. A typical marking could be e.g. Lipemia (turbidity) +++ which means that the patient sample includes a significantly increased amount of e.g. lipids compared to normal patient samples. Automatic analysers like the "Olympus Clinical Chemistry Analyser" identify interference levels of Lipemia (turbidity), Icterus and Hemolysis by using optical measurement of a patient sample and present the levels as shown in Table 1.

**Table 1**

| | **Optical Density** | **Lipemia (Turbidity)** | **Icterus** | **Hemolysis** |
|---|---|---|---|---|
| Increasing level | OD = 0 | + | + | + |
| of interference | ↓ | ++ | ++ | ++ |
| based on | | +++ | +++ | +++ |
| judgement cri- | | ++++ | ++++ | ++++ |
| teria | OD = 2.5 | +++++ | +++++ | +++++ |

Presently the information on the chromatic status of the patient sample is presented to the physician on the patient record together with the results of the different tests performed for the target substances of interest.

Table 2 shows an example for such a patient record. The record shows only one target substance, namely Test A. Usually records include a number of results for different target substances, which are presented together with information on the chromatic interference status. The letter N in Table 2 is used to indicate that there is no chromatic interference.

**Table 2**

| | **Test A** | **Lipemia (Turbidity)** | **Icterus** | **Hemolysis** |
|---|---|---|---|---|
| **Sample 1** | 6.8 | ++ | N | + |
| **Sample 2** | 5.9 | N | +++ | N |
| **Sample 3** | 5.7 | N | N | N |

The physician then has to check in a further table whether or not the actual chromatic interference status of the patient sample could have had an influence on the results of one or more of the different tests performed on the patient sample.

In this context reference is made to Table 3 which shows possible interferences for a number of different target substances. In the first line of the Table 3 the interfering substances (Lipemia = Lipids, Lipoproteins and protein precipitates, Icterus = Bilirubin, Haemolysis = hemoglobin) are indicated. The left column gives the different concentration ranges of each of the substances.

Depending on the nature of the interfering substance and its concentration range, different tests are affected. One can take from Table 3, that the measured concentration for Test A in Sample 1 shown in Table 2, is influenced by the estimated Lipemia (turbidity) interference while the also detected Hemolytic value of this sample has no influence on that test.

Sample 2 has a significant interference level of Icterus measured for Test A and this has to be taken into account when reviewing the results for that sample. Sample 3 has no interference level measured for Lipemia (turbidity), Icterus or Hemolysis.

**Table 3**

| **LIH Warning Flag** | **Lipemia (Turbidity)** | **Icterus** | **Haemolysis** |
|---|---|---|---|
| **+** | **Test A** | **Test A** | **Test C** |
| | **Test B** | **Test H** | **Test D** |
| | **Test C** | **Test I** | **Test Q** |
| | | **Test J** | **Test R** |
| **++** | | | |
| | **Test D** | **Test B** | **Test H** |
| | **Test E** | **Test E** | **Test S** |
| | | **Test K** | **Test T** |
| | | **Test L** | |
| **+++** | | | |
| | **Test U** | **Test D** | **Test U** |
| | **Test F** | **Test H** | **Test V** |
| | | **Test M** | |
| | | **Test N** | |
| | | **Test U** | |
| **++++** | | | |
| | **Test G** | **Test O** | **Test A** |
| | **Test H** | **Test P** | **Test H** |
| | | | **Test I** |
| | | | **Test W** |
| | | | **Test X** |
| **+++++** | **Test V** | **Test Z** | **Test G** |
| | **Test M** | | **Test E** |
| | **Test Q** | | **Test B** |

In view of the high number of patient samples usually processed in laboratories, a more accurate and prompt method is required to indicate possible influence of chromatic substances on the result.

The problem addressed by the invention therefore is to provide a method which is easier to perform which can be automated and which is reliable.

Like known methods also the method according to the invention measures levels of different target substances in a patient sample. Suited patient samples can be any body fluids tissue sample typically used in routine clinical chemistry. In most cases the samples analysed are blood (serum) or urine samples.

The term patient mainly refers to human patients. However, it is understood that the methods addressed to in this application can also be used to analyse tissue originating from animals.

Like in known methods the patient sample is analysed photometrically for the presence of chromatic interfering substances in order to establish its chromatic interfering status. This is performed automatically by addition of a defined portion of patient sample to a defined portion of solution which is suitable for detection of the chromatic interfering substances. These two solutions are mixed and the analysis for interfering chromatic substances is done photometrically at wavelengths specific for the interfering substances in question.

As a rule analysis to establish chromatic interfering substances is performed as the first test for the patient sample and the remaining requested tests for that patient sample are subsequently processed by the analyser. However, it is also possible to perform the analysis at a later stage in the method of the invention. The only requirement is that the chromatic interfering status of the patient sample is available in time to be able to check whether or not the test result for a target substance is influenced by an interfering substance, before presenting the test results to a user.

In one preferred embodiment of the invention discussed more in detail later, it is possible to assess the chromatic interfering status together with the level of a target substance by using the corresponding test sample for this target substance.

Test samples are prepared by mixing of a defined portion of the patient sample with diagnostic reagents specific for at least one of the target substances and selected to create an optical signal depending on the level of target substance.

The test samples are incubated for a time sufficient to allow the formation of an optical signal in the mixture. Then the optical characteristics of the test mixture at at least one wavelength are measured photometrically and based on the values measured and on calibration data established for each of the target substances, the level of the target substances is calculated as test result.

In contrast to known methods the invention provides that the test results are presented to a user together with a marking on the presence of one or more chromatic interfering substances interfering with a test result.

Photometric analysis of the chromatic status of a patient sample as provided by the invention has 2 main advantages.

The first advantage is that compared to visual inspection a more precise objective quantification of the interfering substance is possible.

Further when the method is performed on an automatic analyser, the resulting detected chromatic status of the patient sample can be directly processed by the CPU of the analyser. This allows in a very easy manner that the interference information is presented to a user by directly marking the test results influenced by the interfering substance, as indicated in the following example in Table 4, showing a preferred embodiment of the invention:

### Sample Test Information Presented By The CPU After Analysis

**Table 4**

| **Test Name** | **Result + Attached Flag (if necessary)** |
|---|---|
| Test A | 22 i |
| Test K | 542 i |
| Test U | 5.4 h |
| Test F | 2.3 |
| LIP | N |
| ICT | ++ |
| HEM | +++ |

In contrast to known methods in which a physician only gets information on the chromatic status of the patient sample as a whole, the method according to the invention provides that test results will be marked with an appropriate flag, which indicates that the test result is potentially affected by the presence of an interfering substance. As shown by the above example a physician is not only informed on the concentrations of the different target substances measured (Test A, Test K, Test F and Test U) and the chromatic interfering status of the patient sample. Additionally the result for Test U is marked with the letter h, which means that the increased hemolytic value in the patient sample interferes with the measurement of Test U while Test A and Test K similarly indicates that the result is potentially influenced by a certain detected level of Icterus.

This additional information eliminates the manual review.

Marking of the test results can be done by different manners. A preferred embodiment of the invention provides that marking of the result is done as shown in the above example by adding an information on the interfering substance detected and when required also on its concentration range.

However, the affected test results can also be highlighted in further different manners.

In this preferred method, a more comprehensive LIH flagging feature is provided to improve the customer interpretation of patient test data, as shown in Table 5.

**Table 5**

| **Interference Substance** | **LIH Flag** |
|---|---|
| Lipemia (Turbidity) | 11,12,13,14,15 |
| Icterus | i1, i2, i3, i4, i5 |
| Hemolysis | h1, h2, h3, h4, h5 |

The judgment OD criteria for each level (+ to ++, ++ to +++, etc) will be identified in the L, I or H flag presentation for each individual sample. However, with this preferred embodiment, the relevance of the LIH interference performance for the individual test result according to Table 3 can be directly indicated, allowing a more accurate indication of the influence of L, I or H for the patient sample.

The results of theprevious example in Table 4 would then appear as shown in Table 6.

### Sample Test Information Presented By The CPU After Analysis

**Table 6**

| **Test Name** | **Result + Attached Flag (if necessary)** |
|---|---|
| Test A | 22 i2 |
| Test K | 542 i1 |
| Test U | 5.4 h1 |
| Test F | 2.3 |
| LIP | N |
| ICT | ++ |
| HEM | +++ |

As stated above the rule is that the photometric analysis of the chromatic status is done in a separate assay.

However, as provided in one embodiment of the invention it is also possible to measure the chromatic status of the patient sample in a test sample together with the level of a target substance(s). As the test sample anyhow includes an amount of the patient sample this embodiment saves a separate assay without involving additional effort. The only requirement is that the wavelength used for the test sample in question is different from the wavelengths used to measure the chromatic interfering substances.

As stated above the interfering substances analysed are haemoglobin (haemolyses), lipids, lipoproteins as well as protein precipitates (lipemia) and bilirubin (icterus). The patient sample analysed preferentially is of human origin.

Furthermore in a preferred embodiment all steps of the method according to the invention is performed by an automatic analyser. The results can be displayed on a monitor provided in the analyser.

## Claims

1. Method for measuring the levels of different target substances in a patient sample, especially a body fluid sample,
- in which a defined portion of the patient sample is mixed with a solution which is suitable for detection of chromatic interfering substances
- in which the patient sample is analysed photometrically for the presence of chromatic interfering substances and as a result of the analysis a chromatic interfering status is established for the patient sample,
- and in which test samples are prepared by mixing a defined portion of the patient sample with diagnostic reagents specific for at least one of the target substances and capable to create an optical signal depending on the level of target substance,
- the optical signal in the test sample is measured photometrically for each target substance,
- based on the optical signals measured photometrically, the level of the target substances is determined as test result,
- and the test results are presented to a user,
**characterised in**
**that** before presentation of the test results to the user, it is evaluated for each target substance, whether or not the corresponding test result is influenced by an interfering substance in the patient sample and an appropriate flag, relative to the detected interfering substance, is assigned to those tests which may be influenced by the interfering substance and then the test results and possible flags are presented together to the user.

2. Method according to claim 1, **characterised in that** the marking of the test results is done by presenting the result of the test together with an indication on the kind of the interfering substance.

3. Method according to claim 2, **characterised in that** the marking of the test results additionally includes an indication on the measured level of the interfering substance.

4. Method according to claim 3, **characterised in that** the marking of the level of chromatic interfering substance for the diagnostic test is based on the known chromatic interference performance for the specific test.

5. Method according to claim 1, **characterised in that** the interfering substances analysed are haemoglobin, Lipemia (turbidity) like lipids, lipoproteins as well as protein precipitates and bilirubin.

6. Method according to claim 1, **characterised in that** the patient sample is a human sample, especially a blood, plasma, serum, CSF or urine sample.

7. Method according to claim 1, **characterised in that** the test results are presented by a print out.

8. Method according to claim 1, **characterised in that** the test results are presented by a LIMS or Data Management System.

9. Method according to claim 1, **characterised in that** the method is performed on an automatic analyser.

10. Method according to claim 1, **characterised in that** the presence of chromatic interfering substances is analysed by using one of the test samples.
